# EUROPÄISCHE PATENTSCHRIFT

(11) **EP 0 289 029 B1**
(45) Veröffentlichungstag und Bekanntmachung des Hinweises auf die Patenterteilung: **10.07.1996**
(21) Anmeldenummer: 88106853.0
(22) Anmeldetag: 28.04.1988
(51) Int. Cl.: C08F 2/22, C08F 291/00, C08F 212/08, C08F 4/02, C12N 11/08, C07K 17/08, B01J 39/20, B01J 41/14

(54) **Verfahren zur Herstellung monodisperser Polymerkügelchen**
Process for the preparation of monodisperse polymer particles
Procédé de préparation de particules de polymères monodispersées

(30) Priorität: 29.04.1987 DE 3714258
(43) Veröffentlichungstag der Anmeldung: 02.11.1988
(73) Patentinhaber: Bayer, Ernst, Prof. Dr., 72076 Tübingen (DE)
(72) Erfinder: Bayer, Ernst, Prof. Dr., D-7400 Tübingen (DE); Rapp, Wolfgang, Dr., D-7400 Tübingen (DE)
(74) Vertreter: Kinzebach, Werner, Dr.

(56) Entgegenhaltungen:
- DATABASE WPIL, Nr. 86-262010, Derwent Publications Ltd, Londen, GB; & JP-A-61 190 504 (JAPAN SYNTHETIC RUBBER) 25-08-1986
- DATABASE WPIL, Nr. 84-118557, Derwent Publications Ltd, Londen, GB; & JP-A-59 058 014 (TOKUYAMA SODA) 03-04-1984

## Beschreibung

Die Erfindung betrifft ein Verfahren zur Herstellung monodissperser Polymerkügelchen.

Aufgrund ihres weitgehend inerten Verhaltens und der verfahrenstechnisch einfachen Handhabung finden organische polymere Träger eine immer umfangreichere Anwendung.

Bei chromatographischen Verfahren, wie beispielsweise der Ionenaustauschchromatographie, der Affinitätschromatographie und der Gelpermeationschromatographie werden vor allem modifizierte organische Träger verwendet. Der typische Korngrößenbereich liegt zwischen 5 und 40 µm. Für organische Synthesen, wie beispielsweise der Nucleotid-und Peptidsynthese, werden Trägermaterialien von 30-100 µm Durchmesser benutzt. Handesübliche Ionenaustauscher sind teilsweise noch größer im Durchmesser. Zur Immobilisierung von Enzymen sind Träger mit hoher Oberfläche, d.h. geringen Korngrößen, vorteilhaft (z.B. Latices mit 50-500 nm).

Bei all diesen Trägern handelt es sich in der Regel um polydisperse Materialien, d.h. Materialien mit einer breiten Korngrößenverteilung. Polymere Träger mit einheitlicher, d.h. monodisperser Korngröße, die im allgemeinen um nicht mehr als ± 5 % schwankt, bieten gegenüber den polydispersen Systemen eindeutige Vorteile. Da die Polymerkugel den eigentlichen Reaktionsraum darstellt, erhält man bei monodispersem Material definierte, einheitliche Reaktionsräume. Diffusionszeiten und Verweilzeiten sind in jeder Polymerperle gleich. Gleiche Korngrößen ermöglichen bei chromatographischem Einsatz der Träger optimale Packungen. Neben der Einheitlichkeit der Polymerpartikel spielt die Korngröße der Träger eine bedeutende Rolle. Je größer der Radius ist, desto langsamer verlaufen Diffusionsprozesse im Polymer und damit sinkt die Trennwirksamkeit bei Anwendungen in der Chromatographie. Außerdem ist bei kleinen Durchmessern die verfügbare Oberfläche relativ größer.

Träger mit einer Korngrößenverteilung von 50-1000 µm sind verfahrenstechnisch leicht durch Emulsionspolymerisation zugänglich. Träger mit einer Korngröße von 50-500 µm, sogenannte Latices, lassen sich durch die unterschiedlichsten Verfahren gewinnen. Die Herstellung von Trägern mit einer Korngröße im Bereich von 0,5 - 50 µm, insbesondere von 0,5 - 20 µm, die auf Grund ihrer verfahrenstechnisch einfachen Handhabung besonderes Interesse verdienen, bereitet jedoch Schwierigkeiten.

Es gibt einige Verfahren, die monodisperse Polkymere mit einer Korngröße in diesem interessanten Bereich liefern. So wird in der Literatur beispielsweise ein Verfahren zur Herstellung von Polystyrol-Polymeren in Alkoholen mit Korngrößen von 1 - 5 µm beschrieben. Diese monodispersen Träger tragen funktionelle Gruppen, sind jedoch nicht vernetzt. Ihr Anwendungsgebiet beschränkt sich daher auf Medien, die Polystyrol nicht lösen. Die mechanische Stabilität der unvernetzten Polymermatrix ist gering.

Die EP-A-003 905 beschreibt ebenfalls ein Verfahren zur Herstellung von monodispersem Trägermaterial. Bei diesem Verfahren stellt man zunächst eine Dispersion von Polymerteilchen in Wasser her, indem man ein Monomer in Gegenwart einer sehr schwer wasserlöslichen Verbindung polymerisiert oder diese sehr schwer wasserlösliche Verbindung in Wasser in das aus dem Monomer erhaltenen Polymerisat diffundieren läßt. Hierfür ist es erforderlich, die sehr schwer wasserlösliche Substanz in möglichst feiner Verteilung in das Reaktionsgemisch einzubringen.

In einer zweiten Stufe, die ebenfalls in wäßrigem Medium durchgeführt wird, läßt man dann teilweise wasserlösliche Monomere in die Polymerteilcheneindiffundieren und polymerisiert das so erhaltene Gemisch dann aus. Das in der erwähnten europäischen Patentanmeldung beschriebene Verfahren ist insbesondere aufgrund der ersten Stufe sehr kompliziert und oftmals nicht reproduzierbar. Die JP-A-61 190 504 beschreibt ein analoges Verfahren.

Der Erfindung liegt daher die Aufgabe zugrunde, ein Verfahren zur Herstellung monodisperser Polymerkügelchen zur Verfügung zu stellen, das die Herstellung von Polymerkügelchen mit einem Durchmesser im Bereich von 0,5 bis 50 µm in einfacher und zuverlässiger Weise erlaubt.

Weiter soll das Verfahren ermöglichen, Polymerkügelchen mit variierbaren Eigenschaften herzustellen. Beispielsweise soll es möglich sein, Polymerkügelchen mit beliebiger Porosität, vernetzt oder unvernetzt und mit oder ohne funktionelle Gruppen herzustellen.

Es wurde nun gefunden, daß diese Aufgabe gelöst wird, wenn man eine Polymersaat in einem organischen Lösungsmittel in Gegenwart bestimmter organischer Verbindungen herstellt.

Die Erfindung betrifft daher ein Verfahren zur Herstellung monodisperser Polymerkügelchen mit einem Durchmesser im Bereich von 0,5 bis 50 µm, wobei man in einer ersten Stufe eine Polymersaat herstellt und in einer zweiten Stufe eine Emulsionspolymerisation durchführt, indem man ein Monomer in Gegenwart der Polymersaat, eines Emulgators und eines Polymerisationsinitators und gegebenenfalls eines Vernetzungsmittels in einem wäßrigen Medium zur Polymerisation bringt, das **dadurch gekennzeichnet** ist, daß man in der ersten Stufe ein Monomer in einem Alkohol in Gegenwart von Triphenylmethan oder einer Verbindung der Formel R - X, worin
- R: einen gesättigten oder ungesättigten, geradkettigen oder verzweigten aliphatischen Rest mit mehr als 6 Kohlenstoffatomen bedeutet und
- X: für ein Wasserstoff- oder Halogenatom, eine Hydroxygruppe, C₁-C₆-Alkoxygruppe, Aminogruppe, C₁-C₆-Alkylaminogruppe, Di-C₁-C₆-älkylaminogpuppe, Phenylgruppe oder eine Phenylgruppe bedeutet, welche durch eine C₁-C₄-Alkylgruppe, eine Hydroxygrüppe, welche polyoxyethyleniert sein kann, eine C₁-C₄-Alkoxygruppe, eine Aminogruppe oder eine sulfonsäuregruppe substituiert sein kann, und in Gegenwart eines Polymerisationsinitiators in homogener Phase zur Polymerisation bringt und die erhaltene Polymersaat abtrennt.

Erfindungsgemäß wird die Polymersaat in der ersten Stufe in einem Alkohol, das eine monodisperse Saat erzeugt, hergestellt. Vorzugsweise handelt es sich dabei um geradkettige oder verzweigte C₁-C₆-Alkohole, am bevorzugtesten sind C₁-C₄-Alkohole, wie Methanol, Ethanol und t-Butanol. Bei dem Alkohol kann es sich um einem einzelnen Alkohol oder um ein Gemisch mehrerer Alkohol handeln.

Der Alkohol kann auch im Gemisch mit einem Lösungsmittel, das nicht zur Bildung einer monodispersen Saat führt, verwendet werden. Dieses andere Lösungsmittel dient zur Modifizierung des Lösungsverhaltens. Durch den Zusatz derartiger anderer Lösungsmittel kann man die Teilchengröße der gebildeten Polymersaat steuern. Bei Verwendung eines derartigen anderen Lösungsmittels erhält man eine Polymersaat mit einem größeren Teilchendurchmesser. Der Anteil des anderen Lösungsmittels beträgt im allgemeinen bis zu 20 Volumenprozent, vorzugsweise 5 bis 15 Volumenprozent.

Geeignete Lösungsmittel, die dem eine monodisperse Saat erzeugenden Alkohol zugesetzt werden können, sind insbesondere Kohlenwasserstoffe, wie Pentan und Hexan; aromatische Kohlenwasserstoffe, wie Toluol, o-, m- und p-Xylol; chlorierte Kohlenwasserstoffe, wie Methylenchlorid und Chloroform; Ketone, wie Aceton und Methylethylketon; Ester, wie Essigester; und Ether, wie Tetrahydrofuran und Dioxan. Auch Wasser kann zugesetzt werden, solange die Mischung homogen bleibt. Im allgemeinen kann man maximal 10 Volumenprozent Wasser zusetzen in Abhängigkeit von der Art des organischen Lösungsmittels, der Verbindung R - X und des Monomeren.

Als weiterer Bestandteil für die Herstellung der Polymersaat wird eine Verbindung der Formel R - X zugegeben. Dabei steht R für einen gesättigten oder ungesättigten, geradkettigen oder verzweigten Alkylrest mit mehr als 6 Kohlenstoffatomen und X bedeutet ein Wasserstoff- oder Halogenatom (insbesondere ein Chlor- oder Bromatom), eine Hydroxy- oder C₁-C₆-Alkoxygruppe. eine Amino-C₁-C₆-Alkylamino- oder Di-C₁-C₆-Alkylaminogruppe, eine Phenylgruppe oder eine Phenylgruppe, welche durch eine C₁C₄-Alkylgruppe, eine Hydroxygruppe, die gegebenenfalls polyoxyethyleniert ist, eine C₁-C₄-Alkoxygruppe, eine Aminogruppe oder eine Sulfonsäuregruppe substituiert ist.

Vorzugsweise verwendet man eine Verbindung der Formel R - X, worin R einen gesättigten, vorzugsweise geradkettigen Alkylrest mit 7 bis 20 Kohlenstoffatomen, insbesondere 9 bis 18 Kohlenstoffatomen bedeutet. Beispiele für derartige Reste R sind die geradkettigen C₉-, C₁₀-, C₁₂-, C₁₄-, C₁₆- und C₁₈-Alkylreste. Es können jedoch auch Mischungen dieser Reste vorliegen.

Vorzugsweise steht in der Formel R - X der Rest X für ein Halogenatom (insbesondere ein Chloratom) oder eine Hydroxy-, Amino-, Phenyl- oder substituierte Phenylgruppe. Besonders bevorzugt bedeutet X ein Halogenatom oder eine Phenylgruppe.

Beispiele besonders bevorzugter Verbindungen R - X sind Nonylbenzol oder Dodecylchlorid. Die Verwendung von Triphenylmethan ist ebenfalls bevorzugt, weil sich die Polymersaat bei Verwendung dieser Verbindung besonders einfach handhaben läßt.

Die Verwendung von Verbindungen der Formel R - X, worin R einen ungesättigten Rest bedeutet, ist dann von Vorteil, wenn man die Herstellung von vernetzten monodispersen Polymerkügelchen wünscht.

Bei den in der ersten Stufe für die Herstellung der Polymersaat verwendeten Monomeren kann es sich ganz allgemein um Vinylmonomere handeln. Es können jedoch auch zwei oder mehrere Monomere verwendet werden, so daß man ein Copolymerisat erhält. Vorzugsweise setzt man Styrol oder funktionalisiertes Styrol, beispielsweise durch Chlormethyl-, Sulfonsäure- oder Aminogruppen funktionalisiertes Styrol, ein. Weitere geeignete Monomeren sind Acrylsäurederivate, wie Acrylsäure, Acrylamid, Acrylnitril, und die entsprechenden Methacrylsäurederivate. Die Herstellung der Polymersaat erfolgt in Gegenwart eines Polymerisationsinitiators, bei dem es sich um Verbindungen handelt, die üblicherweise auf diesem Gebiert zur Anwendung kommen. Geeignete Beispiele sind Azoisobutyronitril (AIBN) und Peroxide, wie Benzoylhydroperoxide und Benzoylperoxid.

Weiter kann man für die Herstellung der Polymersaat übliche Hilfsstoffe verwenden, beispielsweise Schutzkolloide, wie Polyvinylpyrrolidon (Molekulargewicht etwa 1 000 000) und Polyvinylalkohol.

Der Anteil an Monomer und der Verbindung R - X ist abhängig von der Art dieser Komponenten und von verwendeten Lösungsmittel. Im allgemeinen jedoch liegt das Verhältnis von Monomer : Verbindung R - X im Bereich von 1:0,1 bis 1:3.

Die Menge an Lösungsmittel wählt man im allgemeinen so, daß etwa 10 bis 20 Gew.-% Monomer im Lösungsmittel enthalten sind.

Die Temperatur für die Herstellung der Polymersaat wählt man im allgemeinen in Abhängigkeit von Lösungsmitteln. Sie liegt im allgemeinen im Bereich von 50 bis 100 °C, vorzugsweise 60 - 90 °C. Die Reaktionszeit kann bis zu 48 Stunden betragen, vorzugsweise liegt sie im Bereich von 24 bis 48 Stunden.

Man erhält auf die oben beschriebene Weise eine monodisperse Polymerszaat mit einer Teilchengröße im Bereich von 0,5 bis 10 µm, vorzugsweise 1 bis 5 µm. Man kann die Teilchengröße auch durch die Menge an Verbindung der Formel R - X steuern. Je größer der Anteil dieser Verbindung ist, umso größer ist die Teilchengröße der Polymersaat.

Die auf diese Weise erhaltene Polymersaat wird dann auf übliche Weise abgetrennt und isoliert, beispielsweise durch Zentrifugieren.

In der zweiten Stufe des erfindungsgemäßen Verfahrens führt man eine Emulsionspolymerisation unter Anwendung der erhaltenen Polymersaat durch. Die Polymerisation erfolgt in wäßrigem Medium unter Zusatz eines Emulgators und eines Polymerisationsinitiators. Als Emulgator kommt einer der auf diesem Gebiet üblichen Emulgatoren zur Anwendung, beispielsweise eine Alkylsulfonsäure, wie Hexadecansulfonsäure oder das Produkt mit der Handelsbezeichnung K-30 (C₁₂-C₁₄-Sulfonsäure) der BASF.

Auch als Poilymerisationsinitiator, der öllöslich sein muß, kommt eine auf diesem Gebiet übliche Verbindung zur Anwendung, beispielsweise eine der oben im Zusammenhang mit der ersten Stuffe genannten Verbindungen.

Bei dem hier zur Anwendung kommenden Monomer handelt es sich allgemein um eine polymerisierbare Vinylverbindung, die auch bifunktionell (zur Vernetzung) sein kann. Vorzugsweise handelt es sich um die Monomeren, die oben im Zusammenhang mit der ersten Stufe beschrieben wurden. Das in der ersten und in der zweiten Stufe verwendete Monomer kann gleich oder verschieden sein.

Für die Herstellung vernetzter Polymerkügelchen verwendet man in der zweiten Stufe auch ein Vernetzungsmittel. Für diesen Zweck können übliche Vernetzungsmittel, wie Divinylbenzol, und bifunktionelle Acrylverbindungen, eingesetzt werden, eine Vernetzung kann jedoch auch dadurch erfolgen, daß man funktionalisierte Monomere zur Anwendung bringt. Ein bevorzugtes funktionalisiertes Monomer ist chlormethyliertes Styrol. Eine Vernetzung erfolgt dann durch Zugabe eines Friedel-Crafts-Katalysators, wie Aluminiumchlorid.

Um die Struktur der monodispersen Polymerkügelchen zu variieren, kann man in der zweiten Stufe auch eine inerte Komponente, die nicht polymerisierbar ist, einsetzen. Diese inerte Komponente muß so beschaffen sein, daß sie in die Polymersaat diffundiert und nach Beendigung der Polymerisation durch geeignete Maßnahmen, wie Auswaschen, Verdampfen durch Erhitzen oder im Vakuum, wieder entrfert werden kann.

Durch den Zusatz der inerten Komponente läßt sich vor allem die Porosität der Polymerkügelchen steuern. Wenn man als Inertkomponente z. B. ein Lösungsmittel für das Polymerisat (Polymersaat) verwendet, erhält man mikroporöse Polymerkügelchen. Wenn man dagegen z. B. eine inerte Komponente verwendet, die kein Lösungsmittel für das Polymerisat darstellt, erhält man makroporöse Polymerkügelchen.

Geeignete Inertkomponenten sind höhere aliphatische, cyclische oder aromatische Kohlenwasserstoffe, wie Heptan, Octan, Cyclohexan, Benzol, Toluol, o-, m-, p-Xylol und Ethylbenzol; höhere Alkohole, wie Hexanol, Heptanol, Octanol, Decanol; cyclische Alkohole, wie Cyclohexanol; chlorierte Kohlenwasserstoffe, wie Dichlormethan und Chloroform. Man kann die inerte Komponente einzeln oder in Mischung zugeben. Die Wahl eines geeigneten Lösungsmittels richtet sich nach dem jeweiligen Polymerisat. Durch die Auswahl eines geeigneten Lösungsmittels oder eines geeigneten Lösungsmittelsgemisches ist es daher möglich, die Lösungseigenschaften der inerten Komponente und somit die Porosität der Polymerkügelchen beliebig zu variieren. Es ist möglich, auf diese Weise Polymerkügelchen mit einer Porengröße von etwa 5 Å bis etwa 3000 Å herzustellen.

Für die Emulsionspolymerisation der zweiten Stufe verwendet man die Polymersaat in einer Menge von 1 bis 20 Gew.-%, vorzugsweise 1 - 15 Gew.-%, bezogen auf die Menge an Monomer.

Als Vernetzungsmittel kommen 0,1 bis 60 Gew.-%, bezogen auf die Menge an Monomer, und je nach gewünschtem Vernetzungsgrad zur Anwendung. Vorteilhafterweise kommt dabei 60 %iges Divinylbenzol in Ethylvinylbenzol zur Anwendung.

Die in das wäßrige Medium eingebrachten Komponenten diffundieren in die Polymersaat. Die Polymersaatpartikel liegen also im Monomer gequollen vor. Die Polymerisation erfolgt dann in den Polymersaatpartikeln.

Man führt die Polymerisation bei einer Temperatur bis maximal 100 °C, vorzugsweise bei 60 bis 90 °C durch. Die Polymerisationszeit beträgt im allgemeinen bis zu 48 Stunden, vorzugsweise liegt sie im Bereich von 24 bis 48 Stunden.

Das erfindungsgemäße Verfahren ergibt also auf einfache Weise monodisperse Polymerkügelchen mit einem Teilchendurchmesser im Bereich von 0,5 bis 50 µm, vorzugsweise im Bereich von 1 bis 20 µm und insbesondere bevorzugt im Bereich von 1 bis 10 µm. Die Polymerkügelchen können dabei vernetzt oder unvernetzt sein. Die Porosität kann je nach Verfahrensbedingungen gewählt werden.

Die erfindungsgemäß erhältlichen Polymerkügelchen haben daher ein breites Anwendungsgebiet. Sie lassen sich insbesondere zur Immobilisierung von Proteinen (Enzymen) und Zellen (für ganze Zellen), als Träger für Katalysatoren (Biokatalysatoren, organische oder anorganische Katalysatoren) oder als Träger für die Peptidsynthese verwenden. Für die Peptidsynthese kommen insbesondere Polymerkügelchen zur Anwendung, die ein Gel bilden, das heißt niedervernetzte Polymerkügelchen mit einem Vernetzungsgrad von 1 bis 5 %, vorzugsweise 1 bis 2 %.

Besonders geeignet sind die erfindungsgemäßen monodispersen Polymerkügelchen zur Anwendung in der Chromatographie. sie finden insbesondere in der Hochdruckflüssigkeitschromatographie (HPLC), Gelpermeationschromatographie, Affinitätschromatographie, Ionenaustauschchromatographie und Ausschlußchromatographie Anwendung.

Es zeigen:
- Fig. 1: ein HPLC-Chromatogramm, erhalten mit erfindungsgemäßen Polymerkügelchen als stationäre Phase,
- Fig. 2: ein HPLC-Chromatogramm, erhalten mit landesüblichen Polymerkügelchen als stationäre Phase,
- Fig. 3: ein weiteres HPLC-Chromatogramm, erhalten mit erfindungsgemäßen Polymerkügelchen als stationäre Phase.

Die nachfolgenden Beispiele erläutern die Erfindung.

### Beispiele

### Beispiel 1

In 480 ml Ethanol werden 384 mg Azoisobutyronitril (AIBN) und 2,4 g Polyvinylpyrolidinon (PVP) gelöst. Zu der Lösung gibt man 30 ml Styrol und 30 ml Dodecylchlorid. Polymerisationstemperatur ist 60° C. Nach 24 Stunden Reaktionszeit erhält man einen monodispersen Polystyrol-Latex mit einer Korgröße von 1,8 um, der ca. 12 % Dodecylchlorid enthält.
Feststoffgehalt: 1 ml Polystyrol/ 10 ml Lösung = 635 mg Latex.

### Beispiel 2

In 24 ml Ethanol werden 12,2 mg AIBN und 120 mg PVP gelöst. Man setzt 1,5 ml Styrol und 2,4 ml Dodecylchlorid zu und erwärmt auf 60° C. Nach 16 Stunden wird nochmals eine Lösung von 2 ml Styrol und 50 mg AIBN in 2 ml Ethanol zugesetzt. Nach weiteren 5 Stunden Reaktionszeit werden 50 mg PVP in 5 ml Ethanol zugesetzt und weitere 24 Stunden polymerisiert. Man erhält monodisperses Polystyrol mit einer Korngröße von 2,5 µm. Die Polymersaat enthält 12 % Dodecylchlorid.

### Beispiel 3

10 ml der in Beispiel 2 hergestellten Saat-Lösung werden 20 Minuten bei 2000 U/min zentrifugiert und die organische Phase abdekandiert. Zu dem Rückstand wird eine wässrige Emulsion von 250 mg Alkylsulfonsäure, 0,4 ml Divinylbenzol, 3 ml Chloromethylstyrol, 17 ml Styrol und 250 mg BPO (Benzoylperoxid) in 180 ml Wasser zugegeben. Man rührt mit 120 U/min 24 Stunden bei Raumtemperatur, und anschließend weitere 48 Stunden bei 72 °C. Die so erhaltenen monodispersen Copolymeren haben einen Durchmesser von 7,8 µm und einen Chlorgehalt von 4 % = 1,14 meq Cl/g.

### Beispiel 4

Man löst 250 mg Emulgator (Alkylsulfonsäure) in 165 ml Wasser und emulgiert darin 3,3 ml Chloromethylstyrol, 4 ml Divinylbenzol, 14,5 ml Styrol und 250 mg BPO. Aus 30 ml Lösung des in Beispiel 1 erzeugten Latex wird wie in Beispiel 3 der Feststoff isoliert und mit der wässrigen Emulsion der Monomeren versetzt.
Man rührt 18 Stunden bei RT mit 100 U/min, erhöht dann die Temperatur auf 72° C. Nach 48 Stunden erhält man ein monodisperses Produkt mit einem Chlorgehalt von 4,47 % = 1,27 meq Cl/g. Die Korngröße beträgt 4,2 µm.

### Beispiel 5

In 48 ml Ethanol werden 38.4 g AIBN und 240 mg PVP gelöst. Man setzt 3 ml Styrol und 3 ml Nonylbenzol zu, spült mit Argon und erwärmt 24 h auf 72°C. Der monodisperse Polystyrol-Latex hat eine Korngröße von 2 µm.

### Beispiel 6

Aus 10 ml der in Beispiel 5 hergestellten Saat-Lösung wird der Polymeranteil durch Abzentrifugieren gewonnen. Man erzeugt eine Emulsion, bestehend aus 16 ml Wasser, 110 mg Alkylsulfonsäure, 8.5 ml Styrol, 2.6 ml Divinylbenzol/Ethylvinylbenzol, 2.2 ml Chloromethylstyrol und 160 mg BPO. Man verzetzt die Polymersaat mit der Monomerenemulsion, rührt 24 h bei Raumtemperatur und erwärmt dann unter Inertgas auf 75°C. Nach 48 h Raktionszeit erhält man einen monodispersen Träger mit einer Korngröße von 5.9 µm. Chlorgehalt: 1.25 meq/g.

### Beispiel 7

Man erzeugt sich eine Monomerenemulsion, bestehend aus 83 mg BPO, 1.1 ml Chloromethylstyrol, 1.3 ml Divinylbenzol/Ethylvinylbenzol, 4.2 ml Styrol, 8.3 ml Wasser und 83 mg Alkylsulfonsäure. Die weitere Umsetzung erfolgt analog Beispiel 6. Man erhält einen monodispersen Träger mit einer Korngröße von 4.5 µm. Chlorgehalt: 1.26 meq/g.

### Beispiel 8

In 100 ml Methanol werden 500 mg PVP und 80 mg AIBN gelöst. Zu der Lösung gibt man6,25 ml Styrol und 6,25 ml Dodecylbenzol, spült mit Argon und erwärmt unter Rühren 48h auf 62°C.Man erhält einen monodispersen Latex mit einer Korngröße von 2,5 µm. Der Latex enthält 46% Dodecylbenzol.

Man isoliert den Latex durch Zentrifugieren und versetzt mit einer Monomeren-Emulsion aus 940 mg Emulgator K-30, 62,5 ml Wasser, 10,8 ml Divinylbenzol/Ethylvinylbenzol (60/40), 48.2 ml Styrol und 800 mg Benzoylperoxid. Die Emulsion wird im Ultraschallbad homogenisiert. Man läßt zwei Stunden bei Raumtemperature eindiffundieren, erhöht die Temperatur auf 80 °C und behält diese Temperature 48 Stunden lang bei. Man erhält auf diese Weise Polystyrolbeads mit einer Korngröße von 8,5 µ.

### Beispiel 9

In 100 ml Ethanol werden 500 mg PVP und 80 mg AIBN gelöst. Man versetz mit 7,2 ml Styrol und6,25 ml Stearylalkohol, spült mit Argon und polymerisiert 48h bei 75° C. Der Latex hat eine Korngröße von 1 µm.

Man behandelt den Latex dann wie in Beispiel 8 beschrieben und erhält so Polystyrolbeads mit einer Korngröße von 2,5 µ.

### Beispiel 10

In 100 ml t-Butanol löst man 500 mg PVP und 80 mg AIBN.Man versetz mit 6,25 ml Styrol und 3.3g Triphenylmethan, spült mit Argon und erwärmt unter Rühren auf 77°C. Der monodisperse Latex hat 1,5 µm Durchmesser.

Man behandelt den Latex dann wie in Beispiel 9 beschrieben und erhält so Polystyrolbeads mit einer Korngröße von 4,8 µ.

### Beispiel 11

Zu einer Lösung aus 100 ml t-Buthanol, 500 mg PVP und 80 mg AIBN werden 12,5 ml Dodecylbenzol und 6,25 ml Styrol gegeben. Nachdem mit Argon gespült wurde, erhitzt man auf 75°C und hält diese Temperatur 48 h. Die Saat hat eine Korngröße von 4 um und einen Gehalt an Dodecylbenzol von 50 %.

Man behandelt den Latex da nn wie in Beispiel 9 beschrieben und erhält so Polystyrolbeads mit einer Korngröße von 9,2 µm.

### Beispiel 12

Zu einer Lösung aus 25 ml Ethanol, 20 mg AIBN, 125 mg Polyvinylalkohol (PVA) gibt man 5 ml THF und 1,56 ml Styrol. Man erwärmt 40 h auf 70°C und erhält so einen 5 µm -Latex.

### Beispiel 13

Zu einer Lösung, bestehend aus 60 ml Ethanol, 6,4 ml Wasser, 300 mg PVP und 48 mg AIBN gibt man 7,5 ml Styrol und die selbe Menge Dodecylchlorid. Man erwärmt 48 h auf 84°C. Man erzeugt so einen Latex von 750 nm.

Man behandelt den Latex dann wie in Beispiel 9 beschrieben und erhält so Polystyrolbeads mit einer Korngröße von 2,2µm.

### Beispiel 14

600 ml Ethanol werden mit 3 g PVP und 480 mg AIBN versetzt. Zu der klaren Lösung gibt man 75 ml Dodecylchlorid und 75 ml Styrol. Man erwärmt 48 h auf 72°C. Korngröße des monodispersen Latex beträgt 2,2 µm.

### Beispiel 15

Aus 120 ml der in Beispiel 14 beschriebenen Latex-Suspension wird der Polystyrol-Latex durch zentrifugieren isoliert.(9,9 g Latex ).
Der Saatlatex wird mit einer feinteiligen Emulsion, bestehend aus 160 ml Wasser, 2,2 g Alkysulfonsäure (K-30), 16 ml Divinylbenzol/Ethyvinylbenzol (60/40 , 64 ml Styrol, 80 ml Heptan und 1 g BPO versetzt.Die Emulsion wird durch Ultraschallbehandlung erzeugt.
Man läßt 30 Min. bei Raumtemperatur unter Rühren eindiffundieren, versetzt dann mit 600 ml Wasser, spült mit Argon und erhitzt 45 h auf 80 °C.
Man erhält so ein makrokporöses vernetztes Polymerisat mit einer Korngroße von 5,5 µm.

### Beispiel 16

Man stellt einen Saatlatex her wie im Beispiel 15 beschrieben. Der Saatlatex wird mit einer feinteiligen Emulsion , bestehend aus 160 ml Wasser, 2 g Alkylsulfonsäure (K-30), 128 ml Divinylbenzol/Ethylvinylbenzol (60/40), 32 ml Styrol und 2,0 g Benzoylperoxid versetzt. Die Emulsion wird durch Ultraschallbehandlung erzeugt. Man läßt 45 Minuten bei Raumtemperatur unter Rühren eindiffundieren, versetzt dann mit 600 ml Wasser, spült mit Argon und erhitzt 45 Stunden auf 80 °C. Man erhält so ein homogen vernetztes Polymerisat mit einer Korngröße von 4,5 µm.

### Beispiel 17

In den nachfolgenden Versuchen wurden erfindungsgemäß erhaltene Polymerkügelchen (MOPS 33/4; Polystyrol/40 % Divinylbenzol; erhalten gemäß Beispiel 16 ) als stationäre Phase in der HPLC-Chromatographie verwendet. Zum Vergleich wurden auch zwei handelsübliche Produkte als stationäre Phase eingesetzt, nämlich ACT-1 (Handelsprodukt der Firma Interaction; Polystyrol, modifiziert mit C₁₈-Alkyl) und PRP (handelsübliche polystyrol-reverse Phase). Die Phasen wurden unter folgenden Bedingungen eingesetzt:

### MOPS 33/4

| | |
|---|---|
| Mobile Phase: | Acetonitril/Wasser 85/15 |
| Korngröße: | 4,5 µm |
| Säule: | 60 x 4,5 |
| Flow: | 1 ml/min beziehungsweise 2 ml/min |
| Druck: | 32 bar |

### ACP-1 und PRP

| | |
|---|---|
| Mobile Phase: | 85 %iges Methanol |
| Flow: | 6 cm/min |
| Säule: | 125 x 4,6 |

Die Detektion erfolgte in allen Fällen bei 254 nm.

Es wurde folgendes Produktgemisch aufgetrennt:
1 N,N-Diethylanilin
2 N,N-Dimethylanilin
3 N-Methylanilin
4 Anilin

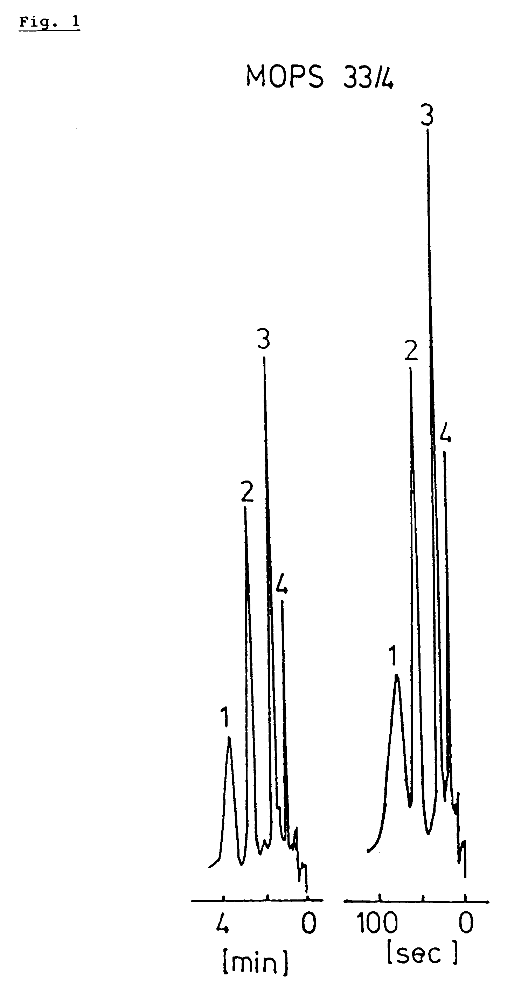

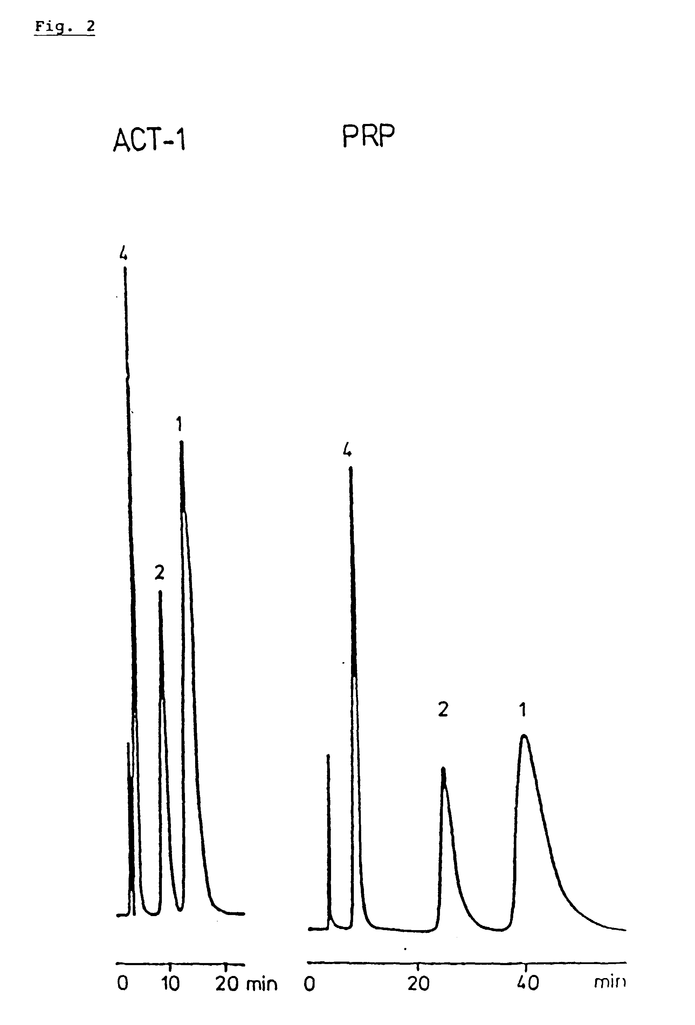

Die erhaltenen Chromatogramme sind in der Figuren 1 und 2 zusammengestellt. Es ist ersichtlich, daß die erfindungsgemäßen Phasen (siehe Figur 1) eine wesentlich höhere Trennwirksamkeit besitzen, da sie trotz einer erheblich kürzeren Säule eine gute Auftrennung ergeben. Außerdem ist bei Verwendung der erfindungsgemäßen Phasen die Peak-Symmetrie deutlich verbessert, während die Phasen des Standes der Technik ein ausgeprägtes Tailing zeigen.

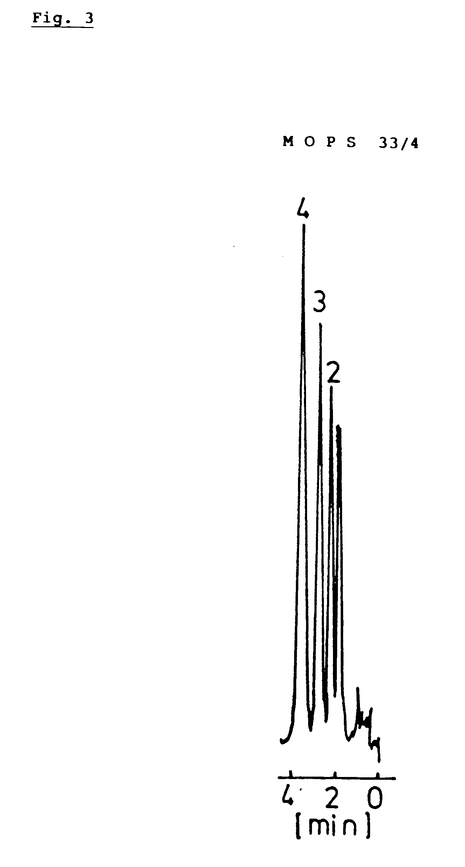

In Figure 3 ist ein Chromatogramm dargestellt, das bei Verwendung der gleichen erfindungsgemäßen Phase unter den oben angegebenen Bedingungen zur Auftrennung des folgenden Produktgemisches erhalten wurde:
1 Toluol
2 Ethylbenzol
3 Propylbenzol
4 Butylbenzol

## Patentansprüche

1. Verfahren zur Herstellung monodisperser Polymerkügelchen mit einem Durchmesser im Bereich von 0,5-50 µm, wobei man in einer ersten Stufe eine Polymersaat herstellt und in einer zweiten Stufe eine Emulsionspolymerisation durchführt, indem man ein Monomer in Gegenwart der Polymersaat, eines Emulgators und eines Polymerisationsinitators und gegebenenfalls eines Vernetzungsmittels in einem wäßrigen Medium zur Polymerisation bringt,
**dadurch gekennzeichnet**, daß man in der ersten Stufe ein Monomer in einem Alkohol in Gegenwart von Triphenylmethan oder einer Verbindung der Formel R - X, worin
R einen gesättigten oder ungesättigten, geradkettigen oder verzweigten aliphatischen Rest mit mehr als 6 Kohlenstoffatomen bedeutet und
X für ein Wasserstoff- oder Halogenatom, eine Hydroxygruppe, C₁-C₆-Alkoxygruppe, Aminogruppe, C₁-C₆-Alkylaminogruppe, Di-C₁-C₆-alkylaminogruppe, Phenylgruppe oder eine Phenylgruppe bedeutet, welche durch eine C₁-C₄-Alkylgruppe, eine Hydroxygruppe, welche polyoxyethyleniert sein kann, eine C₁-C₄-Alkoxygruppe, eine Aminogruppe oder eine Sulfonsäuregruppe substituiert sein kann, und in Gegenwart eines Polymerisationsinitiators in homogener Phase zur Polymerisation bringt und die erhaltene Polymersaat abtrennt.

2. Verfahren nach Anspruch 1, dadurch gekennzeichnet, daß man als Alkohol einen C₁-C₄-Alkohol, verwendet.

3. Verfahren nach Anspruch 2, dadurch gekennzeichnet, daß man als Alkohol Methanol, Ethanol oder t-Butanol verwendet.

4. Verfahren nach einem der vorhergehenden Ansprüche, dadurch gekennzeichnet, daß man den Alkohol im Gemisch mit Wasser, Tetrahydrofuran oder Dioxan verwendet.

5. Verfahren nach einem der vorhergehenden Ansprüche, dadurch gekennzeichnet, daß man in der ersten Stufe eine Verbindung R - X verwendet, in der R einen C₇- C₂₀-Alkylrest, insbesondere einen C₉ - C₁₈-Alkylrest, bedeutet.

6. Verfahren nach einem der vorhergehenden Ansprüche, dadurch gekennzeichnet, daß man in der ersten Stufe eine Verbindung der Formel R - X verwendet, in der X ein Halogenatom oder eine Phenylgruppe bedeutet.

7. Verfahren nach Anspruch 6, dadurch gekennzeichnet, daß man als Verbindung R - X Nonylbenzol oder Dodecylchlorid verwendet.

8. Verfahren nach einem der vorhergehenden Ansprüche, dadurch gekennzeichnet, daß man in der ersten Stufe und/ oder der zweiten Stufe als Monomer Styrol oder ein funktionalisiertes Styrol, insbesondere chlormethyliertes Styrol, oder ein Gemisch davon, verwendet.

9. Verfahren nach einem der vorhergehenden Ansprüche, dadurch gekennzeichnet, daß man in der zweiten Stufe eine inerte, nicht polymerisierbare Komponente zusetzt.

10. Verwendung der nach einem der Ansprüche 1 bis 9 erhaltenen monodispersen Polymerkügelchen als Träger für die Immobilisierung von Proteinen und Zellen, als Träger für Katalysatoren, für die Peptidsynthese und für die Chromatographie.

## Claims

1. A process for preparing monodispersed polymer beads having a diameter within the range of 0.5-50 µm, in which a polymer seed is prepared in a first stage and an emulsion polymerisation is carried out in a second stage by polymerising a monomer in an aqueous medium, in the presence of the polymer seed, an emulsifier, a polymerisation initiator and, optionally, a crosslinking agent, characterised in that, in the first stage, a monomer is polymerised in an alcohol, in a homogenous phase, in the presence of triphenyl methane or a compound of formula R - X, wherein
R is a saturated or unsaturated, straight-chained or branched aliphatic group having more than 6 carbon atoms and
x is a hydrogen or halogen atom, a hydroxy group, C₁₋₆-alkoxy group, amino group, C₁₋₆-alkyl amino group, di-C₁₋₆-alkyl amino group, phenyl group or a phenyl group which may be substituted by a C₁₋₄-alkyl group, a hydroxy group which may be polyoxyethylenated, a C₁₋₄-alkoxy group, an amino group or a sulphonic acid group, in the presence of a polymerisation initiator, and the polymer seed obtained is separated.

2. A process according to claim 1, characterised in that the alcohol used is a C₁₋₄-alcohol.

3. A process according to claim 2, characterised in that methanol, ethanol or t-butanol is used as the alcohol.

4. A process according to one of the preceding claims, characterised in that the alcohol is used in admixture with water, tetrahydrofuran or dioxane.

5. A process according to one of the preceding claims, characterised in that, in the first stage, a compound R - X is used in which R is a C₇₋₂₀-alkyl group, more particularly a C₉₋₁₈-alkyl group.

6. A process according to one of the preceding claims, characterised in that, in the first stage, a compound of formula R - X is used in which X is a halogen atom or a phenyl group.

7. A process according to claim 6, characterised in that nonylbenzene or dodecyl chloride is used as compound R - X.

8. A process according to one of the preceding claims, characterised in that, in the first stage, and/or the second stage, styrene or a functionalised styrene, more particularly chloromethylated styrene or a mixture thereof, is used as the monomer.

9. A process according to one of the preceding claims, characterised in that an inert, non-polymerisable component is added in the second stage.

10. Use of the monodispersed polymer beads, obtained according to one of the claims 1 to 9, as substrates for the immobilisation of proteins and cells, as substrates for catalysts, in peptide synthesis and chromatography.

## Revendications

1. Procédé de préparation de particules de polymères monodispersées ayant un diamètre allant de 0,5 à 50 µm,
dans lequel, dans une première étape, on prépare des termes de polymère et, dans une seconde étape, on procède à une polymérisation en émulsion en mettant un monomère,en présence des germes de polymère, d'un émulsifiant et d'un initiateur de polymérisation et éventuellement d'un agent de réticulation, dans un milieu aqueux pour la polymérisation,
caractérisé par le fait que, dans la première étape, on met en phase homogène pour la polymérisation un monomère dans un alcool en présence de triphénylméthane ou d'un composé de formule R - X, dans laquelle
R désigne un radical aliphatique, saturé ou non saturé, à chaîne linéaire ou ramifiée, avec plus de 6 atomes de carbone et
X désigne un arome d'hydrogène ou d'halogène, un groupe hydroxy, un groupe C₁-C₆-alkoxy, un groupe amino, un groupe C₁-C₆-alkylamino, un groupe di-C₁-C₆-alkylamino, un groupe phényle ou un groupe phényle substitué par un groupe C₁-C₄-alkyle, un groupe hydroxy, qui peut être polyoxyéthyléné, un groupe C₁-C₄-alkoxy, un groupe amino ou un groupe acide sulfonique, et en présence d'un initiateur de polymérisation et on sépare les germes de polymère obtenus.

2. Procédé selon la revendication 1, caractérisé par le fait que, comme alcool, on utilise un C₁-C₄-alcool.

3. Procédé selon la revendication 2, caractérisé par le fait que, comme alcool, on utilise du méthanol, de l'éthanol ou du t-butanol.

4. Procédé selon l'une des revendications précédentes, caractérisé par le fait que l'on utilise l'alcool en mélange avec de l'eau, du tétrahydrofuranne ou du dioxanne.

5. Procédé selon l'une des revendications précédentes, caractérisé par le fait que l'on utilise, dans la première étape, un composé R-X, dans lequel R représente un radical C₇-C₂₀-alkyle, en particulier un radical C₉-C₁₈-alkyle.

6. Procédé selon l'une des revendications précédentes, caractérisé par le fait que, dans la première étape, on utilise un composé dc formule R-X dans laquelle X désigne un atome d'halogène ou un groupe phényle.

7. Procédé selon la revendication 6, caractérisé par le fait que, comme composé R-X, on utilise le monylbenzène ou le chlorure de dodécyle.

8. Procédé selon l'une des revendications précédentes, caractérisé par le fait que, dans la première étape et/ou la seconde étape, on utilise comme monomère le styrène ou un styrène fonctionnel, en particulier le styrène chlorométhylé ou un mélange de ces produits.

9. Procédé selon l'une des revendications précédentes, caractérisé par le fait que, dans la deuxième étape, on ajoute un composant inerte, non polymérisable.

10. Utilisation des particules de polymère monodispersées obtenues selon l'une des revendications 1 à 9 comme support pour l'immobilisation de protéines et de cellules, comme support pour catalyseurs, pour la synthèse de peptides et pour la chromatographie,
